**Europäisches Patentamt**

**European Patent Office**

**Office européen des brevets**

(19)

(11) **EP 1 393 767 A1**

(12) **EUROPÄISCHE PATENTANMELDUNG**

(43) Veröffentlichungstag:
**03.03.2004 Patentblatt 2004/10**

(51) Int Cl.⁷: **A61M 16/00**

(21) Anmeldenummer: **03015231.8**

(22) Anmeldetag: **05.07.2003**

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(30) Priorität: **20.08.2002 DE 10237973**

(71) Anmelder: **Gottlieb Weinmann Geräte für Medizin und Arbeitsschutz GmbH + Co.**
**22525 Hamburg (DE)**

(72) Erfinder: **Tiemann, Björn**
**22926 Ahrensburg (DE)**

(74) Vertreter: **Klickow, Hans-Henning**
**Patentanwälte**
**Hansmann-Klickow-Hansmann**
**Jessenstrasse 4**
**22767 Hamburg (DE)**

(54) **Verfahren und Vorrichtung zur Erfassung eines Strömungsvolumens**

(57) Das Verfahren und die Vorrichtung dienen zur Erfassung eines Strömungsvolumens bei der Durchführung eines Beatmungsvorganges. Es wird mindestens ein mit dem Strömungsvolumen im Zusammenhang stehender Parameter meßtechnisch erfaßt und einer Auswertungseinheit zugeführt. Das Strömungsvolumen wird von einer Pumpeinrichtung (13) gefördert, die von einem elektrisch gespeisten Motor (14) angetrieben ist. Es wird sowohl eine Drehzahl des Motors (14) als auch ein Druck im Bereich des Strömungsvolumens meßtechnisch erfaßt und der Auswertungseinheit zugeführt. Das Strömungsvolumen wird von der Auswertungseinheit durch eine rechnerische Verknüpfung der Meßwerte für den Druck und die Drehzahl ermittelt. Zur meßtechnischen Erfassung des Druckes und der Drehzahl werden Sensoren verwendet.

FIG.2

EP 1 393 767 A1

**Beschreibung**

Die Erfindung betrifft ein Verfahren zur Erfassung eines Strömungsvolumens bei der Durchführung eines Beatmungsvorganges, bei dem mindestens ein mit dem Strömungsvolumen im Zusammenhang stehender Parameter meßtechnisch erfaßt und einer Auswertungseinheit zugeführt wird und bei dem das Strömungsvolumen vom einer Pumpeinrichtung gefördert wird, die von einem elektrisch gespeisten Motor angetrieben ist.

Die Erfindung betrifft darüber hinaus eine Vorrichtung zur Erfassung eines Strömungsvolumens bei der Durchführung eines Beatmungsvorganges, die mindestens einen Sensor zur Messung mindestens eines mit dem Strömungsvolumen im Zusammenhang stehenden Parameters aufweist und bei der der Sensor an eine Auswertungseinheit angeschlossen ist sowie bei der eine Pumpeinrichtung zur Förderung des Strömungsvolumens mit einem elektrisch gespeisten Motor versehen ist.

Bei der Durchführung einer Beatmung von Patienten ist es häufig erforderlich, eine möglichst exakte Erfassung des Strömungsvolumens an Atemgas durchzuführen. Eine derartige Erfassung ist sowohl im Hinblick auf eine Überwachung der Beatmungseinrichtung als auch im Hinblick auf eine Steuerung der Beatmungseinrichtung von Bedeutung. Im Hinblick auf die Überwachungsfunktion ist es durch die Erfassung des Strömungsvolumens an Beatmungsgas möglich, eine ausreichende Sauerstoffversorgung des Patienten zu kontrollieren und gegebenenfalls entsprechende Anzeigeeinrichtungen zu aktivieren oder gegebenenfalls durch die Steuerung des Gerätes ein vergrößertes oder vermindertes Strömungsvolumen bereitzustellen. Bei einer Steuerung des Gerätes ist insbesondere auch eine Synchronisierung der Funktion des Beatmungsgerätes mit einem natürlichen Atmungsrhythmus des Patienten von Bedeutung, damit der Patient nicht gegen das Beatmungsgerät anatmen muß und hierdurch eine zusätzliche Atemarbeit für den Patienten verursacht wird.

Zur Durchführung einer Ermittlung des Strömungsvolumens ist es beispielsweise bekannt, Sensoren mit temperierbaren Hitzdrähten zu verwenden, die mit temperaturabhängigen Widerständen versehen sind. Bei einer Beheizung eines der Widerstandsdrähte kann im Bereich eines weiteren Hitzdrahtes eine Temperaturveränderung erfaßt werden, die vom jeweiligen Strömungsvolumen abhängig ist. Ebenfalls ist es bereits bekannt, über dem Strömungsvolumen ausgesetzte Meßbrücken mit temperaturabhängigen Widerständen das Strömungsvolumen zu erfassen, da auch hier Abkühleffekte vom jeweiligen Strömungsvolumen abhängig sind. Die bekannten Sensoren erweisen sich jedoch als relativ aufwendig und insbesondere bei wechselnden Strömungsrichtungen sowie zeitlich variierenden Strömungsgeschwindigkeiten sind entweder aufwendige Auswertungseinrichtungen erforderlich oder die Meßgenauigkeit kann noch nicht alle Anforderungen erfüllen.

Aufgabe der vorliegenden Erfindung ist es daher, ein Verfahren der einleitend genannten Art derart zu verbessern, daß mit geringem aparativen Aufwand eine genaue Erfassung des Strömungsvolumen unterstützt wird.

Diese Aufgabe wird erfindungsgemäß dadurch gelöst, daß sowohl eine Drehzahl des Motors als auch ein Druck im Bereich des Strömungsvolumens meßtechnisch erfaßt sowie der Auswertungseinheit zugeführt werden und daß das Strömungsvolumen von der Auswertungseinheit durch eine rechnerische Verknüpfung der Meßwerte für den Druck und die Drehzahl ermittelt wird.

Weitere Aufgabe der vorliegenden Erfindung ist es, eine Vorrichtung der einleitend genannten Art derart zu konstruieren, daß eine hohe Meßgenauigkeit bei Verwendung einfacher Sensoren erreicht wird.

Diese Aufgabe wird erfindungsgemäß dadurch gelöst, daß der Motor mit einem Sensor zur Drehzahlerfassung versehen und im Bereich des Strömungsvolumens ein Sensor zur Druckmessung angeordnet ist, daß beide Sensoren an die Auswertungseinheit angeschlossen sind und daß die Auswertungseinheit mit einer Recheneinheit zur Bestimmung des Strömungsvolumens durch eine rechnerische Verknüpfung der Meßwerte für den Druck und die Drehzahl versehen ist.

Die meßtechnische Erfassung der Drehzahl des Motors sowie die meßtechnische Erfassung des Druckes im Bereich des Strömungsvolumens können mit konstruktiv einfachen Sensoren preiswert und mit hoher Genauigkeit erfolgen. Durch die rechnerische Auswertung dieser Meßgrößen im Bereich der Auswertungseinheit ist es möglich, unter Berücksichtigung eines mathematischen Modells der strömungstechnischen Eigenschaften der Gesamtanordnung das jeweilige Strömungsvolumen zu ermitteln. Die Berechnung des Strömungsvolumens aus den gemessenen Parametern erfolgt nach vergleichsweise einfachen Berechnungsvorschriften, so daß die Umrechnung beispielsweise unter Verwendung eines einfachen Mikroprozessors durchgeführt werden kann. Alternativ ist es aber auch möglich, eine analoge Ermittlung mit Hilfe von Bauelementen durchzuführen, die geeignete Kennlinien besitzen.

Eine Ermittlung eines aktuellen Strömungsvolumens wird dadurch unterstützt, daß die rechnerische Verknüpfung unter Berücksichtigung einer Gerätecharakteristik durchgeführt wird, die durch ein Kennlinienfeld definiert wird.

Zur Ermöglichung einer Ermittlung des Strömungsvolumens mit geringem Zeitaufwand wird vorgeschlagen, daß das Kennlinienfeld vor einer Erfassung des Strömungsvolumens meßtechnisch aufgenommen wird.

Eine weitere Verkürzung des Zeitaufwandes kann dadurch erfolgen, daß eine tatsächliche physikalische Abhängigkeit zwischen dem Strömungsvolumen und einem im Bereich des Strömungsvolumens herrschenden Druck durch eine gemischt quadratische Gleichung angenähert wird.

Zur Bereitstellung einfacher Berechnungsvorschriften wird vorgeschlagen, daß eine Parametrisierung des Kennlinienfeldes durchgeführt wird.

Eine weitere Vereinfachung erfolgt dadurch, daß eine Abhängigkeit von Sekundärkoeffizienten der gemischt quadratischen Gleichung von der Drehzahl durch eine gemischt quadratische Gleichung bestimmt wird.

Eine hohe Verarbeitungsgeschwindigkeit wird auch dadurch unterstützt, daß Primärkoeffizienten der Gleichung für die Drehzahlabhängigkeit vor einer Ermittlung des Strömungsvolumens festgelegt und abgespeichert werden.

Zur Bereitstellung aktueller Werte für ein jeweiliges Strömungsvolumen wird vorgeschlagen, daß bei der Erfassung des Strömungsvolumens zyklisch eine Ermittlung der Sekundärkoeffizienten aus den abgespeicherten Primärkoeffizienten in Abhängigkeit von der jeweiligen Drehzahl durchgeführt wird.

Ein typischer Verfahrensablauf erfolgt derart, daß zyklisch zu einer jeweiligen Drehzahl aus den abgespeicherten Primärkoeffizienten die Sekundärkoeffizienten errechnet und unter Verwendung der Sekundärkoeffizienten und unter Berücksichtigung des aktuellen Druckes das aktuelle Strömungsvolumen berechnet wird.

Fig. 1 Eine perspektivische Darstellung eines Beamtungsgerätes mit Verbindungsschlauch zu einer Beatmungsmaske,

Fig. 2 ein prinzipielles Blockschaltbild zur Veranschaulichung einer gerätetechnischen Anordnung der verwendeten Sensoren,

Fig. 3 ein Diagramm zur Veranschaulichung von Kennlinien, die die Abhängigkeit des Strömungsvolumens vom Druck veranschaulichen und für unterschiedliche Motordrehzahlen dargestellt sind,

Fig. 4 ein Diagramm zur Gegenüberstellung eines zeitlichen Verlaufes eines tatsächlichen Strömungsvolumens und eines errechneten Strömungsvolumens.

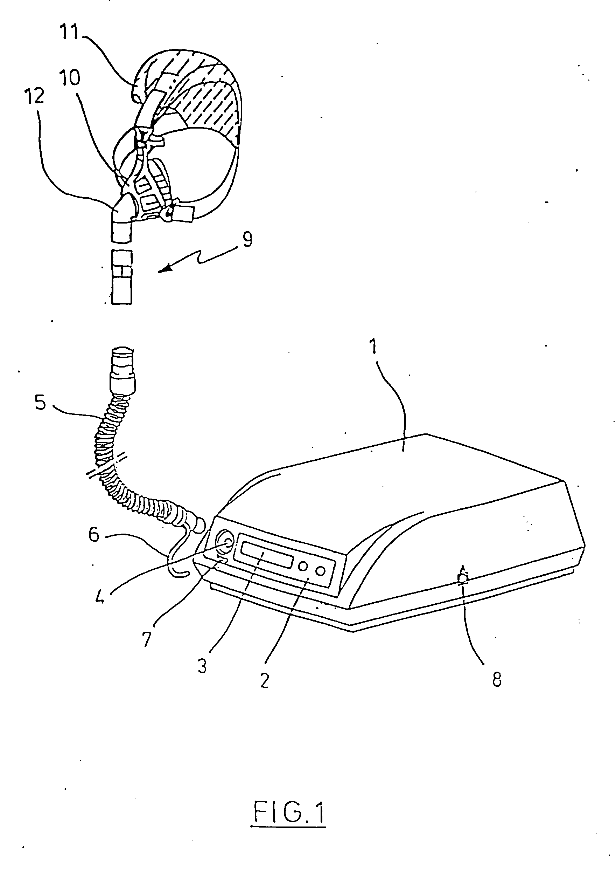

Fig. 1 zeigt den grundsätzlichen Aufbau einer Vorrichtung zur Beatmung. Im Bereich eines Gerätegehäuses (1) mit Bedienfeld (2) sowie Anzeige (3) ist in einem Geräteinnenraum eine Atemgaspumpe angeordnet. Über eine Kopplung (4) wird ein Verbindungsschlauch (5) angeschlossen. Entlang des Verbindungsschlauches (5) kann ein zusätzlicher Druckmeßschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf.

Im Bereich einer dem Gerätegehäuse (1) abgewandten Ausdehnung des Verbindungsschlauches (5) ist ein Ausatmungselement (9) angeordnet. Ebenfalls kann ein Ausatemventil verwendet werden.

Fig. 1 zeigt darüber hinaus eine Beatmungsmaske (10), die als Nasalmaske ausgebildet ist. Eine Fixierung im Bereich eines Kopfes eines Patienten kann über eine Kopfhaube (11) erfolgen. Im Bereich ihrer dem Verbindungsschlauch (5) zugewandten Ausdehnung weist die Beatmungsmaske (10) ein Kupplungselement (12) auf.

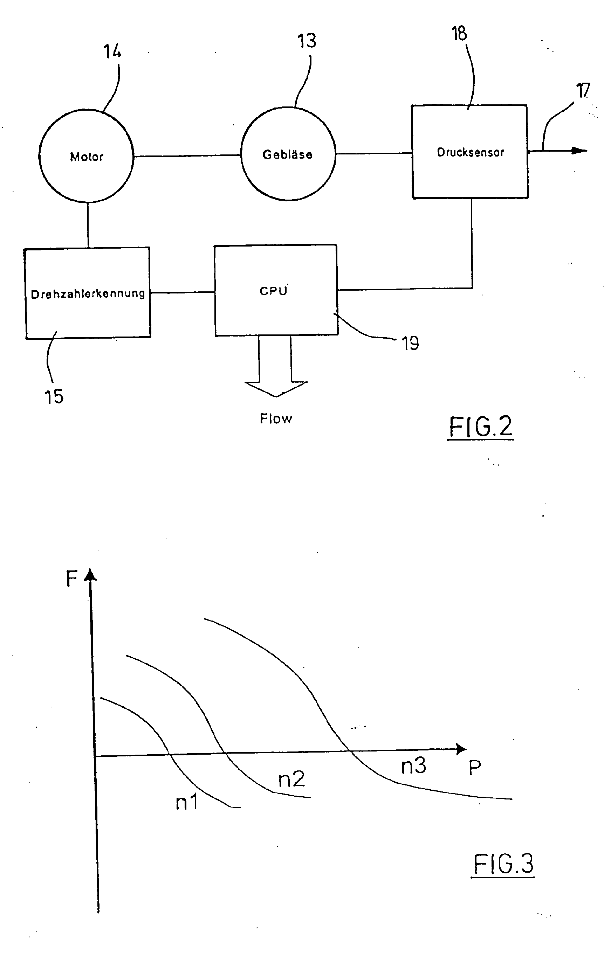

Fig. 2 zeigt ein schematisches Blockschaltbild zur Erläuterung eines Teiles eines inneren Aufbaues des Gerätes entsprechend Fig. 1. Zur Förderung des Strömungsvolumens an Atemgas ist eine Pumpeinrichtung (13) vorgesehen, die als ein Gebläse ausgebildet ist. Die Pumpeinrichtung (13) wird von einem Motor (14) angetrieben. Zur Erfassung einer Drehzahl des Motors (14) ist ein Sensor (15) verwendet, der an eine Auswertungseinheit (16) angeschlossen ist. Im Bereich einer von der Pumpeinrichtung (13) gespeisten Leitung (17) für das Strömungsvolumen ist ein Sensor (18) zur Erfassung eines Druckes angeordnet. Auch der Sensor (18) ist an die Auswertungseinheit (16) angeschlossen.

Im Bereich der Auswertungseinheit (16) ist eine Recheneinheit (19) implementiert, die eine rechnerische Verknüpfung der Meßwerte für den Druck und die Drehzahl vornimmt und hieraus einen aktuellen Volumenfluß berechnet. Bei einer Ausbildung der Auswertungseinheit (16) als Digitalrechner, beispielsweise als Mikroprozessor, ist es möglich, die Recheneinheit (19) als Teil der Ablaufprogrammierung der Auswertungseinheit (16) zu realisieren. Bei einer analogen Ausbildung der Auswertungseinheit (16) ist auch daran gedacht, die Recheneinheit (19) über Bauelemente mit linearem oder nichtlinearem elektrischen Verhalten zu realisieren.

Fig. 3 veranschaulicht ein Kennlinienfeld, das für ein beispielhaft gewähltes Beatmungssystem die Abhängigkeit des Strömungsvolumens vom jeweiligen Druck wiedergibt. Es ist insbesondere zu erkennen, daß sich bei unterschiedlichen Drehzahlen des Motors (14) unterschiedliche Kennlinienverläufe ergeben. Die jeweiligen Kennlinien bestehen aus näherungsweise parabelförmigen Teilbereichen, die in einer Umgebung der Druck-Achse von näherungsweise linearen Verläufen verbunden sind.

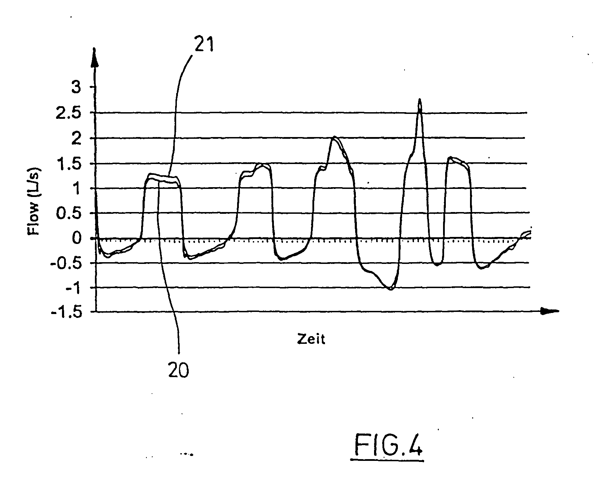

Fig. 4 stellt einen gemessenen Verlauf (20) des Strömungsvolumens und einen von der Auswertungseinheit (16) berechneten Verlauf (21) gegenüber. Das Strömungsvolumen ist dabei in 1/sec angegeben. Es ist zu erkennen, daß eine sehr weitgehende Übereinstimmung vorliegt.

Für eine Realisierung des erfindungsgemäßen Verfahrens sowie bei einem Aufbau der erfindungsgemäßen Vorrichtung wird meßtechnisch das Kennlinienfeld gemäß Fig. 3 bestimmt. Für unterschiedliche Motordrehzahlen wird dabei für die Gesamtheit aus Motor (14), Pumpeinrichtung(13), Leitung (17) sowie Sensor (18) die jeweilige Kennlinie bestimmt. Für die einzelnen Kennlinien K zu jeder Drehzahl ergibt sich hierbei der Druck P in Abhängigkeit vom Volumenfluß F entsprechend der Gleichung

$$P_k(F) = a_{k2}{}^*F^2 + a_{k1}{}^*F + a_{k0}$$

Die obige Gleichung stellt eine Annäherung des tatsächlichen Verlaufes dar, es zeigt sich aber, daß diese Annäherung eine gute Übereinstimmung mit dem tatsächlichen Kennlinienverlauf aufweist. Jeder der Sekundärkoeffizienten $a_{ki}$ mit i = 0 bis 2 ist von der jeweiligen Drehzahl abhängig. Die obige Gleichung läßt sich deshalb auch in der Form

$$P(F) = a_2(n)^* F^2 + a_1(n)^*F + a_0(n)$$

darstellen. Es zeigt sich, daß auch die Sekundärkoeffizienten $a_{ki}$ in guter Näherung mit einer quadratischen Gleichung aus der jeweiligen Drehzahl bestimmt werden können. Ein entsprechender Gleichungsansatz lautet

$$a_i = b_{i2}{}^*n^2 + b_{i1}{}^*n + b_{i0}.$$

Die Primärkoeffizienten $b_{ik}$ repräsentieren hier die mechanischen und elektrischen Eigenschaften des Gesamtsystems und können meßtechnisch bestimmt werden. Unter Berücksichtigung der quadratischen Ansätze für die Abhängigkeit zwischen dem Druck und dem Volumenfluß sowie den Sekundärkoeffizienten $a_{ik}$ von den Primärkoeffizienten $b_{ik}$ sind insgesamt neun Koeffizienten $b_{ik}$ meßtechnisch zu bestimmen.
Die einfache Gleichungsstruktur ermöglicht es, die Sekundärkoeffizienten $a_{k(n)}$ vor jedem neu zu berechnenden Wert für den Volumenfluß aus den bekannten Primärkoeffizienten $b_i$ neu zu berechnen. Der eigentliche Wert für den Volumenfluß wird dann über die Umkehrfunktion der zuerst aufgeführten Gleichung ermittelt.

**Patentansprüche**

1.  Verfahren zur Erfassung eines Strömungsvolumens bei der Durchführung eines Beatmungsvorganges, bei dem mindestens ein mit dem Strömungsvolumen im Zusammenhang stehender Parameter meßtechnisch erfaßt und einer Auswertungseinheit zugeführt wird und bei dem das Strömungsvolumen von einer Pumpeinrichtung gefördert wird, die von einem elektrisch gespeisten Motor angetrieben ist, **dadurch gekennzeichnet, daß** sowohl eine Drehzahl des Motors (14) als auch Druck im Bereich des Strömungsvolumens meßtechnisch erfaßt sowie der Auswertungseinheit (16) zugeführt werden und daß das Strömungsvolumen von der Auswertungseinheit (16) durch eine rechnerische Verknüpfung der Meßwerte für den Druck und die Drehzahl ermittelt wird.

2.  Verfahren nach Anspruch 1, **dadurch gekennzeichnet, daß** die rechnerische Verknüpfung unter Berücksichtigung einer Gerätecharakteristik durchgeführt wird, die durch ein Kennlinienfeld definiert wird.

3.  Verfahren nach Anspruch 1 oder 2, **dadurch gekennzeichnet, daß** das Kennlinienfeld vor einer Erfassung des Strömungsvolumens meßtechnisch aufgenommen wird.

4.  Verfahren nach einem der Ansprüche 1 bis 3, **dadurch gekennzeichnet, daß** eine tatsächliche physikalische Abhängigkeit zwischen dem Strömungsvolumen und einem im Bereich des Strömungsvolumens herrschenden Druck durch eine gemischt quadratische Gleichung angenähert wird.

5.  Verfahren nach einem der Ansprüche 1 bis 4, **dadurch gekennzeichnet, daß** eine Parametrisierung des Kennlinienfeldes durchgeführt wird.

6.  Verfahren nach einem der Ansprüche 1 bis 5, **dadurch gekennzeichnet, daß** eine Abhängigkeit von Sekundärkoeffizienten der gemischt quadratischen Gleichung von der Drehzahl durch eine gemischt quadratische Gleichung bestimmt wird.

7.  Verfahren nach Anspruch 6, **dadurch gekennzeichnet, daß** Primärkoeffizienten der Gleichung für die Drehzahlabhängigkeit vor einer Ermittlung des Strömungsvolumens festgelegt und abgespeichert werden.

8.  Verfahren nach einem der Ansprüche 1 bis 7, **dadurch gekennzeichnet, daß** bei der Erfassung des Strömungsvolumens zyklisch eine Ermittlung der Sekundärkoeffizienten aus den abgespeicherten Primärkoeffizienten in Abhängigkeit von der jeweiligen Drehzahl durchgeführt wird.

9.  Verfahren nach einem der Ansprüche 1 bis 8, **dadurch gekennzeichnet, daß** zyklisch zu einer jeweiligen Drehzahl aus den abgespeicherten Primärkoeffizienten die Sekundärkoeffizienten errechnet und unter Verwendung der Sekundärkoeffizienten und unter Berücksichtigung des aktuellen Druk-

kes das aktuelle Strömungsvolumen berechnet wird.

10. Vorrichtung zur Erfassung eines Strömungsvolumens bei der Durchführung eines Beatmungsvorganges, die mindestens einen Sensor zur Messung mindestens eines mit dem Strömungsvolumens im Zusammenhang stehenden Parameters aufweist und bei der der Sensor an eine Auswertungseinheit angeschlossen ist sowie bei der eine Pumpeinrichtung zur Förderung des Strömungsvolumens mit einem elektrische gespeisten Motor versehen ist, **dadurch gekennzeichnet, daß** der Motor (14) mit einem Sensor (15) zur Drehzahlerfassung versehen und im Bereich des Strömungsvolumens ein Sensor (18) zur Druckmessung angeordnet ist, daß beide Sensoren (15,18) an die Auswertungseinheit (16) angeschlossen sind und daß die Auswertungseinheit (16) mit einer Recheneinheit (19) zur Bestimmung des Strömungsvolumens durch eine rechnerische Verknüpfung der Meßwerte für den Druck und die Drehzahl versehen ist.

11. Vorrichtung nach Anspruch 10, **dadurch gekennzeichnet, daß** die Recheneinheit eine Speichereinrichtung zur Bevorratung mindestens eines Teiles von Kenndaten eines Kennlinienfeldes aufweist, das eine Abhängigkeit des Volumenflusses vom Druck definiert.

12. Vorrichtung nach Anspruch 10 oder 11, **dadurch gekennzeichnet, daß** die Kenndaten des Kennlinienfeldes in einem Kalibrierungsvorgang vor der Durchführung der Strömungserfassung ermittelt und abgespeichert sind.

13. Vorrichtung nach einem der Ansprüche 10 bis 12, **dadurch gekennzeichnet, daß** die Kenndaten des Kennlinienfeldes über Sekundärkoeffizienten einer gemischt-quadratischen Gleichung als Annäherung an einen tatsächlichen Kennlinienverlauf definiert sind.

14. Vorrichtung nach einem der Ansprüche 10 bis 13, **dadurch gekennzeichnet, daß** ein Zusammenhang zwischen den Sekundärkoeffizienten und einer aktuellen Drehzahl durch eine gemischt quadratische Gleichung definiert ist.

15. Vorrichtung nach Anspruch 14, **dadurch gekennzeichnet, daß** Sätze von Primärkoeffizienten zur Bestimmung der Sekundärkoeffizienten unter Berücksichtigung einer aktuellen Drehzahl abgespeichert sind.

16. Vorrichtung nach einem der Ansprüche 10 bis 15, **dadurch gekennzeichnet, daß** die Auswertungseinheit (16) eine Steuerung zur zyklischen Ermittlung der Sekundärkoeffizienten aufweist.

17. Vorrichtung nach Anspruch 16, **dadurch gekennzeichnet, daß** die Steuerung in einem ersten Schritt eine Ermittlung der Sekundärkoeffizienten unter Berücksichtigung einer aktuellen Drehzahl aus den Primärkoeffizienten und in einem zweiten Schritt eine Ermittlung des Strömungsvolumens unter Verwendung der Sekundärkoeffizienten und unter Berücksichtigung des aktuellen Druckes vorgibt.

FIG.1

Motor

Gebläse

Drucksensor

Drehzahlerkennung

CPU

Flow

FIG.2

F

n1

n2

n3

P

FIG.3

FIG.4

## EUROPÄISCHER RECHERCHENBERICHT

**Europäisches Patentamt**

**Nummer der Anmeldung**

EP 03 01 5231

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | WO 96 11717 A (BIRD PRODUCTS CORP) 25. April 1996 (1996-04-25) * Seite 35, Zeile 22 - Seite 37, Zeile 14 * * Seite 38, Zeile 23 - Seite 39, Zeile 10; Abbildungen 1,12 * --- | 1-17 | A61M16/00 |
| X | WO 00 66207 A (GENGER HARALD ;MAP GMBH (DE); NEGELE CLAUS (DE)) 9. November 2000 (2000-11-09) * Seite 4, letzter Absatz - Seite 5, Absatz 19; Abbildungen * --- | 1-3, 10-13 | |
| X | WO 97 10019 A (FINN SHANE DARREN ;RESMED LTD (AU); FARRUGIA STEVEN PAUL (AU)) 20. März 1997 (1997-03-20) * Zusammenfassung; Abbildungen * ----- | 1,10 | |

**RECHERCHIERTE SACHGEBIETE (Int.Cl.7)**

A61M

Der vorliegende Recherchenbericht wurde für alle Patentansprüche erstellt

| Recherchenort | Abschlußdatum der Recherche | Prüfer |
|---|---|---|
| MÜNCHEN | 14. November 2003 | Valfort, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

........................................................

& : Mitglied der gleichen Patentfamilie, übereinstimmendes Dokument

9

**ANHANG ZUM EUROPÄISCHEN RECHERCHENBERICHT
ÜBER DIE EUROPÄISCHE PATENTANMELDUNG NR.**  EP 03 01 5231

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-11-2003

| Im Recherchenbericht angeführtes Patentdokument | Datum der Veröffentlichung | Mitglied(er) der Patentfamilie | Datum der Veröffentlichung |
|---|---|---|---|
| WO 9611717 A | 25-04-1996 | CA 2201698 A1 | 03-10-1998 |
| | | US 2002005197 A1 | 17-01-2002 |
| | | BR 9509306 A | 23-12-1997 |
| | | EP 0800412 A1 | 15-10-1997 |
| | | JP 10507116 T | 14-07-1998 |
| | | WO 9611717 A1 | 25-04-1996 |
| | | US 5694926 A | 09-12-1997 |
| | | US 5881722 A | 16-03-1999 |
| | | US 5868133 A | 09-02-1999 |
| | | AT 235280 T | 15-04-2003 |
| | | DE 69530117 D1 | 30-04-2003 |
| | | EP 1205206 A2 | 15-05-2002 |
| | | EP 1205203 A2 | 15-05-2002 |
| WO 0066207 A | 09-11-2000 | AU 4561400 A | 17-11-2000 |
| | | AU 4916500 A | 17-11-2000 |
| | | DE 10021782 A1 | 16-11-2000 |
| | | DE 10021783 A1 | 30-11-2000 |
| | | DE 10021784 A1 | 30-11-2000 |
| | | DE 20008049 U1 | 17-08-2000 |
| | | WO 0066209 A1 | 09-11-2000 |
| | | WO 0066207 A1 | 09-11-2000 |
| | | EP 1175240 A1 | 30-01-2002 |
| | | FR 2793145 A1 | 10-11-2000 |
| WO 9710019 A | 20-03-1997 | AU 719713 B2 | 18-05-2000 |
| | | AU 6919096 A | 01-04-1997 |
| | | WO 9710019 A1 | 20-03-1997 |
| | | CA 2232083 A1 | 20-03-1997 |
| | | EP 0855923 A1 | 05-08-1998 |
| | | JP 11513268 T | 16-11-1999 |
| | | US 6332463 B1 | 25-12-2001 |

Für nähere Einzelheiten zu diesem Anhang : siehe Amtsblatt des Europäischen Patentamts, Nr.12/82